(19) Europäisches Patentamt
European Patent Office
Office européen des brevets

(11) EP 3 839 473 A1

(12) # EUROPEAN PATENT APPLICATION
published in accordance with Art. 153(4) EPC

(43) Date of publication:
**23.06.2021 Bulletin 2021/25**

(21) Application number: **19850589.3**

(22) Date of filing: **13.08.2019**

(51) Int Cl.:
***G01N 1/00*** *(2006.01)* ***B01J 4/02*** *(2006.01)*
***C12N 5/04*** *(2006.01)* ***C12N 5/07*** *(2010.01)*
***G01N 1/28*** *(2006.01)* ***G01N 35/10*** *(2006.01)*
***C12M 1/00*** *(2006.01)* ***C12M 1/24*** *(2006.01)*
***C12M 1/26*** *(2006.01)* ***C12N 1/04*** *(2006.01)*

(86) International application number:
**PCT/JP2019/031860**

(87) International publication number:
**WO 2020/036180 (20.02.2020 Gazette 2020/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **17.08.2018 JP 2018153635**

(71) Applicant: **Osaka University**
**Suita-shi, Osaka 565-0871 (JP)**

(72) Inventor: **KINOOKA Masahiro**
**Suita-shi, Osaka 565-0871 (JP)**

(74) Representative: **Dehns**
**St. Bride's House**
**10 Salisbury Square**
**London EC4Y 8JD (GB)**

(54) **PARTICLE DISTRIBUTION METHOD**

(57) Provided is a distribution method with which particle components such as cells can be dispensed. In one aspect, provided is a method for distributing particles, the method including dispersing the particles in a plastic fluid, and dispensing the plastic fluid containing the particles. In another aspect, provided is a method for producing a product including a particle and a container for the particle, the method including dispensing a plastic fluid in which the particles are dispersed, from a storage part in which the plastic fluid is stored, to a plurality of the containers.

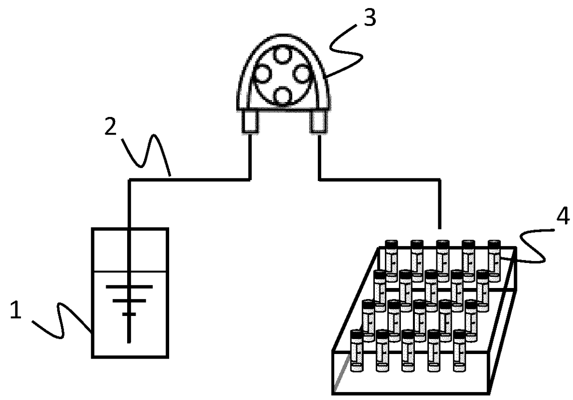


FIG.1

EP 3 839 473 A1

## Description

### Technical Field

The present disclosure relates to distribution of particles, dispensing of fluid containing particles, and manufacture of a product containing particles.

### Background Art

A dispensing operation of ejecting a uniform amount of solution is carried out in, for example, a sampling operation for component analysis, or a vial filling process for filling an industrial product into vials, wherein the principal component of the product is a small amount of solution. The development of an automatic analyzer that automatically performs a dispensing operation has been also developed (Patent Documents 1 and 2).

In a case of a liquid in which substances having largely different densities are mixed, dispersed, or dissolved, such as a mixture of a plurality of types of solutions, or a solution containing microparticles, it is necessary to keep the concentrations of the substances in the liquid to be dispensed constant before the dispensing and after the start of the dispensing (during the dispensing process), in order to dispense the liquid so that the concentrations of the substances are uniform. To solve this problem, a physical stirring operation using magnetic microparticles or ultrasonic waves in a storage part has been proposed (Patent Documents 3 and 4).

### Prior Art Document

### Patent Document

[Patent Document 1] JP-A-2009-058288
[Patent Document 2] JP-A-2012-509485
[Patent Document 3] JP-A-2008-102052
[Patent Document 4] Japanese Patent No. 5668021

## SUMMARY OF THE INVENTION

### Problem to be Solved by the Invention

In the above-described dispensing method with physical stirring, however, it is necessary to optimize the stirring method and parameters of respective operations according to components of samples and the scale.

Besides, in a case of a sample containing a living component such as cells, there are concerns that the activity thereof might deteriorate due to physical stimulation of stirring.

In light of this, the present disclosure, in one aspect, provides a novel distribution method with which particle components such as cells can be dispensed.

### Means to Solve the Problem

In one aspect, the present disclosure relates to a method for distributing particles, the method including:

dispersing the particles in a plastic fluid; and
dispensing the plastic fluid containing the particles.

In one aspect, the present disclosure relates to a producing method for producing a product that includes a particle and a container for the particle, the producing method including:
dispensing a plastic fluid in which the particles are dispersed, from a storage part in which the plastic fluid is stored, to a plurality of the containers.

Further, in another aspect, the present disclosure relates to a composition to be used in dispensing in the distribution method or the producing method according to the present disclosure, the composition containing a substance for converting a liquid in which particles are dispersed, or a liquid that disperses particles, into a plastic fluid.

Still further, in another aspect, the present disclosure relates to a composition containing a cell cryopreservation solution and a substance for converting the cell cryopreservation solution into a plastic fluid.

Effect of the Invention

According to the present disclosure, in one aspect, distribution in which unevenness of the amount (or density) of particles is suppressible without stirring in a storage part during a distributing operation can be provided.

Brief Description of Drawings

[FIG. 1] FIG. 1 schematically illustrates an automated pipetting device.
[FIG. 2] FIG. 2 explains Formula (I).
[FIG. 3] FIG. 3 explains an outline of an experiment scheme.
[FIG. 4] FIG. 4 shows a graph illustrating the relationship between stirring and the density of beads in a vial.
[FIG. 5] FIG. 5 shows graphs illustrating the relationship between stirring and the density of beads in a vial.
[FIG. 6] FIG. 6 shows graphs illustrating the relationship between stirring and a dispensed amount in a vial.
[FIG. 7] FIG. 7 explains an outline of an experiment scheme using cultured cells.
[FIG. 8] FIG. 8 shows graphs illustrating the relationship between stirring and the cell viability (P/Po).
[FIG. 9] FIG. 9 explains an outline of an experiment scheme using a plastic fluid.
[FIG. 10] FIG. 10 shows graphs illustrating the relationship between the use of a plastic fluid and the density of beads.
[FIG. 11] FIG. 11 shows graphs illustrating that a plastic fluid did not have cytotoxicity.
[FIG. 12] FIG. 12 shows photographs illustrating that a plastic fluid did not have cytotoxicity.
[FIG. 13] FIG. 13 shows graphs illustrating exemplary experiment results from which influences of a plastic fluid on the cell potential were confirmed.

Mode for Carrying out the Invention

The present disclosure is based on the following findings: in a case where particles such as cells or beads are dispensed, the use of a plastic fluid can suppress fluctuations in the concentration or density of particles in an aliquot dispensed, even without a stirring operation during the dispensing process.
The present disclosure is also based on the following findings: when dispensing without use of a stirring operation during a dispensing process is made possible, even a living particle component such as cells, or a fragile particle component can avoid damage caused by stirring, while fluctuations in the concentration or density of the particles in an aliquot dispensed can be suppressed.
The present disclosure is further based on the following findings: when dispensing without use of a stirring operation during a dispensing process is made possible, gas contamination (mixing of gas (air bubble) into the liquid) in a storage part (reservoir) can be suppressed, whereby fluctuations in the dispensed amount can be suppressed.
The present disclosure is still further based on the following findings: using a plastic fluid when a solution is dispensed with use of a pump and a tube makes it possible to feed the solution in laminar flow in the tube, thereby making it easier to maintain the same concentration of particles as the concentration during sucking. In addition, the present disclosure is based on the following findings: when feeding the solution in laminar flow is made possible, collapse of interface between the solution and a gas phase is suppressed, which makes it unlikely that the solution would contain gas.
The present disclosure, however, is not limited by these findings.

[Distribution Method, Dispensing Method]

In one aspect, the present disclosure relates to a method for distributing particles, the method including dispersing the particles in a plastic fluid, and dispensing the plastic fluid containing the particles.
In the distribution method according to the present disclosure, for example in a case where the particles are cells, an exemplary plastic fluid is a plastic fluid obtained by mixing a specific compound described below in a medium or a cryopreservation solution.
The distribution method for distributing particles according to the present disclosure, therefore, can be paraphrased as follows. Specifically, in one aspect, the present disclosure relates to a dispensing method that includes making a liquid for a particle-containing liquid into a plastic fluid, and aliquoting the particle-containing plastic fluid.
With the distribution method and the dispensing method of the present disclosure, in one or a plurality of embodiments, a particle-containing liquid (plastic fluid) can be distributed or dispensed uniformly in density and uniformly in amount, without an operation such as a stirring operation. In one or a plurality of embodiments, therefore, the distribution method and the dispensing method of the present disclosure include distributing or dispensing a particle-containing

plastic fluid in aliquots.

The following description describes particles and plastic fluids.

[Particles]

In the distribution/dispensing method of the present disclosure, "particles" refer to those that can be dispensed when mixed in a liquid.

The particles may be in a solid or liquid form.

The particles in one or a plurality of embodiments are cells, microorganisms, liposomes, medicine-containing particles, cosmetic components such as pigments, beads, or colloidal particles.

Since a stirring operation during a distribution/dispensing process is not essential in the distribution/dispensing method of the present disclosure, the method is preferable for distributing/dispensing particles sensitive to shear stress (physical stress). The particles are not necessarily limited to these.

The "cells" can encompass cells derived from animals, and cells derived from plants. The cells in one or a plurality of embodiments are cultured cells. Exemplary cultured cells are stem cells; the stem cells are, for example, pluripotent stem cells such as embryonic stem cells (ES cells) and induced pluripotent stem cells (iPS cells), or multipotential stem cells such as mesenchymal stem cells, hematopoietic stem cells, and tissue stem cells.

Examples of beads include polymer beads, ceramic beads, metal beads, and metal oxide beads. Examples of the bead form include magnetic beads, bead carriers, and abrasive grains.

Examples of colloid include emulsion and suspension.

In the present disclosure, the "distribution of particles" encompasses dispensing of a mixture of particles and a liquid.

In the present disclosure, the "liquid" can mean both of a liquid that is not a plastic fluid, and a plastic fluid. Unless specified otherwise, the "liquid" means a liquid that is not a plastic fluid.

[Plastic Fluid]

In the present disclosure, the "plastic fluid" refers to a fluid that requires a yield stress to be made to flow, that is, a fluid having a yield value. The plastic fluid may be a Bingham fluid, or a non-Bingham fluid. In the present disclosure, the "plastic fluid" refers to a plastic fluid that can suppress the sedimentation of particles to be distributed (dispensed), or preferably can hold particles without sedimentation in a standstill state.

In the present disclosure, in one or a plurality of non-limiting embodiments, the plastic fluid can be obtained by mixing a liquid that is not a plastic fluid and a specific compound.

The specific compound is a compound that can convert a liquid that is not a plastic fluid into a plastic fluid, and in one or a plurality of non-limiting embodiments, compounds disclosed in WO2014/017513 or compounds described below can be used as the specific compound.

The specific compound for converting a liquid that is not a plastic fluid into a plastic fluid is not particularly limited, but it is, for example, a high molecular compound, or preferably a high molecular compound having an anionic functional group.

Examples of the anionic functional group include carboxy group, sulfo group, phosphate group, and salts of these; among these, carboxy group or a salt thereof is preferred.

As the high molecular compound, a high molecular compound having one, two, or more selected from the group consisting of the anionic functional groups can be used.

Preferable specific examples of the specific compound for converting a liquid that is not a plastic fluid into a plastic fluid include, though not particularly limited to these, a polysaccharide in which ten or more monosaccharides (e.g., triose, tetrose, pentose, hexose, or heptose) are polymerized; more preferably, the specific compound is an acidic polysaccharide having an anionic functional group. The "acidic polysaccharide" referred to herein is not particularly limited as long as it has an anionic functional group in its structure. The acidic polysaccharide is, for example, a polysaccharide having an uronic acid (e.g., glucuronic acid, iduronic acid, galacturonic acid, or mannuronic acid), a polysaccharide having a sulfate group or a phosphate group in a part of its structure, or a polysaccharide having both of the structures. The acidic polysaccharide encompasses, not only polysaccharides obtained from nature, but also polysaccharides produced by microorganisms, polysaccharides produced by genetic engineering, and polysaccharides artificially synthesized with use of an enzyme. More specifically, the acidic polysaccharide is, for example, constituted of one, two, or more selected from the group consisting of hyaluronic acid, gellan gum, deacylated gellan gum (hereinafter referred to as DAG in some cases), rhamsan gum, diutan gum, xanthan gum, carrageenan, xanthan gum, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and salts of these. The polysaccharide is preferably hyaluronic acid, DAG, diutan gum, xanthan gum, carrageenan, or a salt of any of these; more preferably the polysaccharide is DAG, considering that when DAG is

used at a low concentration, particles are suspended, and are easily collected. Examples of the salt referred to herein include: salts of alkali metals such as lithium, sodium, and potassium; salts of alkali earth metals such as calcium, barium, and magnesium; and salts of aluminum, zinc, steel, iron, ammonium, organic bases, and amino acids.

These high molecular compounds (polysaccharides) preferably have a weight-average molecular weight of 10,000 to 50,000,000, more preferably, 100,000 to 20,000,000, and further preferably 1,000,000 to 10,000,000. For example, the molecular weight can be measured in terms of pullulan by gel permeation chromatography (GPC). Alternatively, as DAG, phosphorylated DAG can be used. The phosphorylation can be performed by a known method.

In one or a plurality of embodiments, as the polysaccharide, a plurality (preferably two) of the polysaccharides can be used in combination. The type of the combination of polysaccharides is not limited particularly; preferably, the combination includes at least DAG or a salt thereof. More specifically, the preferable combination of polysaccharides includes DAG or a salt thereof, and a polysaccharide other than DAG or a salt thereof (e.g., xanthan gum, alginic acid, carrageenan, diutan gum, methylcellulose, locust bean gum, or a salt of any of these). Examples of a specific combination of polysaccharides include: DAG and rhamsan gum; DAG and diutan gum; DAG and xanthan gum; DAG and carrageenan; DAG and xanthan gum; DAG and locust bean gum; DAG and κ-carrageenan; DAG and sodium alginate; and DAG and methylcellulose, though not limited to these.

Further preferable specific examples of the specific compound include hyaluronic acid, deacylated gellan gum, diutan gum, carrageenan, xanthan gum, and salts of these, among which deacylated gellan gum and salts of the same are more preferable. In the present disclosure, deacylated gellan gum is a linear high molecular weight polysaccharide containing four molecules of saccharides as constituent units, the saccharides being 1,3-bonded glucose, 1,4-bonded glucuronic acid, 1,4-bonded glucose, and 1,4-bonded rhamnose; the polysaccharide is expressed by Formula (I) below, where $R_1$ and $R_2$ each represent a hydrogen atom, and n represents an integer of 2 or more. Here, $R_1$ may include a glyceryl group, and $R_2$ may include an acetyl group; the content of the glyceryl group and the acetyl group is preferably 10% or less, or more preferably 1% or less.

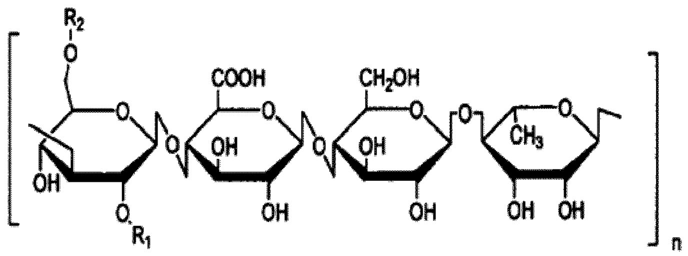

(I)

The mechanism of converting the liquid into a plastic fluid varies with the above-described specific compounds; in the case of deacylated gellan gum, when mixed with a liquid, the deacylated gellan gum takes in metal ions (for example, calcium ions) in the liquid, and forms a structure in an undefined shape with the metal ions being interposed therein, to suspend the particles. In one or a plurality of embodiments, a liquid as a plastic fluid prepared with use of deacylated gellan gum has a viscosity of 8 mPa·s or less, preferably 4 mPa·s or less, and more preferably 2 mPa·s or less considering the collectability of the particles. The viscosity of the liquid as a plastic fluid, however, is not limited particularly, and may exceed the above-described values.

The specific compound to convert the liquid into a plastic fluid can be obtained by chemical synthesis, but in a case where the compound is a natural product, the compound is preferably obtained by extraction and separation/purification with a conventional technique from any of various plants, animals, and microorganisms containing the compound. In the extraction, water or a supercritical gas may be used so that the compound can be extracted efficiently. For example, in the gellan gum producing method, the following may be carried out: microorganisms producing gellan gum are cultured in a fermentation medium, then, a mucous substance produced extracellularly is collected by a common purification method, and dried, crushed, etc., followed by pulverization. In a case of deacylated gellan gum, the following may be carried out: a mucous substance is subjected to an alkali treatment when being collected, and the glyceryl and acetyl groups bound to 1,3-bonded glucose residues are collected after being acylated. Regarding the purification method, for example, any of the following may be used alone, or in combination in an arbitrary order, and repeatedly, so as to remove impurities for purification: liquid-liquid extraction; fractional precipitation; crystallization; various types of ion-exchange chromatography; gel filtration chromatography using sephadex LH-20 or the like; adsorption/desorption of an active substance by adsorption chromatography using activated charcoal, silica gel, etc., or thin-layer chromatography; and high-performance liquid chromatography using a reversed-phase column. Examples of gellan gum producing microorganisms include Sphingomonas elodea, and microorganisms obtained by modifying genes of Sphingomonas elodea, though not limited to these.

In the case of deacylated gellan gum, a commercially available product may be used, which is, for example,

"KELCOGEL (registered trademark of C. P. Kelco) CG-LA" manufactured by Sanshin Co., Ltd., or "KELCOGEL (registered trademark of C. P. Kelco)" manufactured by San-Ei Gen FFI Co., Ltd. Alternatively, "KELCOGEL (registered trademark of C. P. Kelco) HT" manufactured by San-Ei Gen FFI Co., Ltd. can be used as native gellan gum.

[Concentration of Specific Compound in Plastic Fluid]

The concentration of the specific compound in the plastic fluid (mass/volume%, hereinafter simply referred to as "%") is dependent on the type of the specific compound, and can be set appropriately in such a range that the plastic fluid can hold cells in a storage bottle without sedimentation in a standstill state; the concentration of the same is normally 0.0005% to 1.0%, preferably 0.001 % to 0.4%, more preferably 0.005% to 0.1%, and further preferably 0.005% to 0.05%. For example, in a case of deacylated gellan gum, the concentration of the same is 0.001% to 1.0%, preferably 0.003% to 0.5%, more preferably 0.005% to 0.1%, further preferably 0.01% to 0.05%, and still further preferably 0.02% to 0.05%. For example, in a case of xanthan gum, the concentration of the same is 0.001% to 5.0%, preferably 0.01% to 1.0%, more preferably 0.05% to 0.6%, and further more preferably 0.3% to 0.6%. For example, in a case of mixture of κ-carrageenan and locust bean gum, the concentration of the same is 0.001% to 5.0%, preferably 0.005% to 1.0%, more preferably 0.01% to 0.1%, and most preferably 0.03% to 0.05%. In a case of native gellan gum, the concentration of the same is 0.05% to 1.0%, and more preferably 0.05% to 0.1%.

In a case where a plurality of types (preferably two types) of the above-described polysaccharides are used in combination, the concentration of the polysaccharides can be set appropriately in such a range that the plastic fluid can hold cells in the liquid without sedimentation in a standstill state. For example, in a case where a combination of DAG or a salt thereof, and a polysaccharide other than DAG or the salt thereof, is used, an exemplary concentration of DAG or the salt thereof is 0.005 to 0.02%, and preferably 0.01 to 0.02%; and an exemplary concentration of the polysaccharide other than DAG or the salt thereof is 0.005 to 0.4%, and preferably 0.1 to 0.4%. Examples of the combination of specific concentration ranges include the following.

DAG or salt thereof: 0.005 to 0.02% (preferably 0.01 to 0.02%)
Polysaccharide other than DAG
xanthan gum: 0.1 to 0.4%
sodium alginate: 0.1 to 0.4%
locust bean gum: 0.1 to 0.4%
methylcellulose: 0.1 to 0.4% (preferably 0.2 to 0.4%)
carrageenan: 0.05 to 0.1%
diutan gum: 0.05 to 0.1%

The concentration can be calculated by the following formula.

$$\text{Concentration (\%)} = \text{mass of specific compound (g)/volume of liquid (ml)} \times 100$$

The specific compound can be converted to still another derivative by chemical synthesis, and the derivative thus obtained can be also used effectively in such a cell culturing method according to the present disclosure. Specifically, in a case of deacylated gellan gum, a derivative obtained by substituting the hydroxyl group corresponding to $R_1$ and/or $R_2$ of the compound expressed by Formula (I) with the following can also be used in the present invention: a C1-3 alkoxy group; a C1-3 alkylsulfonyl group; a residue of a monosaccharide such as glucose or fructose; a residue of an oligosaccharide such as sucrose or lactose; a residue of an amino acid such as glycine or arginine; etc. It is also possible to crosslink the compound by using a cross-linker such as 1-ethyl-3-(3-di-methylaminopropyl)carbodiimide (EDC).

The specific compound or a salt thereof may exist as an arbitrary crystal form depending on production conditions, and may exist as an arbitrary hydrate. These crystal forms, hydrates, and mixtures thereof are also encompassed in the present invention. The specific compound may also exist as a solvate containing an organic solvent, such as acetone, ethanol, or tetrahydrofuran; all of these forms are encompassed in the present disclosure.

The specific compound may exist in a form of a tautomer or a geometric isomer generated by endocyclic or exocyclic isomerization, or a mixture of tautomers or geometric isomers, or alternatively, a mixture of any of these. In the present disclosure, the specific compound when having an asymmetric center may exist in a form of a resolved optical isomer or a mixture containing them at an arbitrary ratio, irrespective of whether the specific compound is generated by isomerization or not.

In one or a plurality of embodiments, the liquid as a plastic fluid can be produced by adding a solution or a dispersion of the specific compound to a liquid culture medium. As the specific compound aggregates with ions (the compound assembles via ions), or the specific compound forms a three-dimensional network, a metal ion is preferably

added as required. In one or a plurality of embodiments, the metal ion is a divalent metal ion. In one or a plurality of embodiments, examples of the divalent metal ion include calcium ion, magnesium ion, zinc ion, ferrous ion, and copper ion. In one or a plurality of embodiments, the concentration of the metal ion is 0.1 mM to 300 mM, 0.5 mM to 100 mM, or 0.5 mM to 10 mM.

[Yield Value of Plastic Fluid]

In the present disclosure, the yield value of the plastic fluid used in the present disclosure is preferably at such a level that the plastic fluid can hold particles without sedimentation in a standstill state. With a view to holding particles, the plastic fluid used in the present disclosure preferably has a yield value of 0.02 Pa or more, and more preferably, 0.03 Pa or more. The plastic fluid used in the present disclosure has a yield value of, for example, 0.2 Pa or less, or 0.1 Pa or less.

[Automated Pipetting System]

The distribution/dispensing method of the present disclosure can be favorably applied to dispensing by an automated pipetting system. With the distribution/dispensing method of the present disclosure, in one or a plurality of non-limiting embodiments, it is possible to suppress at least one of the unevenness of the dispensed amount for an aliquot, the unevenness of the particle density, and the unevenness of the cell viability in a case where the particles are cells, when the dispensing is performed by the automated pipetting system.

The automated pipetting system is not particularly limited in the present disclosure, and examples of the same include: a type that performs sucking from a storage part (reservoir) with use of a pump unit including a feeding pipe and a pump, and performs ejecting to vials or a multiwell plate; and a type that performs dispensing from a storage part to a multiwell plate with use of a dispensing pipet head that is capable of sucking and ejecting.

In one or a plurality of embodiments, the automated pipetting system of the present disclosure includes a control unit for controlling a pump unit, a dispensing pipet head, and the like. In one or a plurality of embodiments, the control unit controls a pump unit, a dispensing pipet head, and the like, so as to dispense a particles-dispersed plastic fluid stored in a storage part in aliquots into a plurality of containers.

[Producing Method]

In another aspect, the present disclosure relates to a producing method for producing a product including a particle and a container for the particle, the producing method including dispensing a plastic fluid in which the particles are dispersed, from a storage part in which the plastic fluid is stored, to a plurality of the containers.

The producing method of the present disclosure can be described differently as follows. In another aspect, the present disclosure relates to a producing method for producing a product that includes a liquid and a container for the liquid, the producing method including dispensing the liquid from a storage part of the liquid into a plurality of the containers, wherein the liquid is a plastic fluid containing particles.

With the producing method of the present disclosure, in one or a plurality of embodiments, it is possible to produce products having substantially uniform amounts (or concentrations) of particles and/or substantially uniform amounts of liquid. With the producing method of the present disclosure, in one or a plurality of embodiments, it is possible to dispense substantially the same amount (or concentration) of particles and/or the same amount of liquid as that dispensed into a container at the start of dispensing, even when a remaining amount of the plastic fluid or liquid in a storage part arranged in an automated pipetting system becomes 20% or less, 15% or less, 10% or less, 5% or less, 4% or less, 3% or less, 2% or less, or 1% or less of the amount before the start of dispensing. Besides, in a case where the remaining amount of the plastic fluid or liquid in the storage part arranged in the automated pipetting system becomes equal to or less than the dispensing amount, all of the remainder can be dispensed in one container.

In one or a plurality of embodiments, the producing method of the present disclosure includes dispensing a particle-dispersed plastic fluid in aliquots into a plurality of containers. In one or a plurality of particularly non-limiting embodiments, the producing method of the present disclosure includes dispensing the particle-dispersed plastic fluid or liquid stored in a storage part arranged in an automated pipetting system, in aliquots into a plurality of containers. In one or a plurality of embodiments, the producing method of the present disclosure includes producing a plurality of the products dispensed in aliquots, by using an automated pipetting system.

In the present disclosure, "dispensing in aliquots" encompasses dispensing particles/liquid into a plurality of containers in such a manner than the containers contain substantially uniform amounts (or concentration) of the particles and/or substantially uniform amounts of the liquid. In the present disclosure, "substantially uniform amounts (or concentration) of the particles" means that a relative error of an amount (or concentration) ($Y_p$) of particles dispensed in a container with respect to a desired amount (or concentration) ($X_p$) of the particles ($\{(X_p - Y_p)/X_p\} \times 100$) is $\pm$15% or less,

±14% or less, ±13% or less, ±12% or less, ±11% or less, ±10% or less, ±9% or less, ±8% or less, ±7% or less, ±6% or less, ±5% or less, ±4% or less, ±3% or less, ±2% or less, ±1% or less, or ±0.55% or less. In the present disclosure, "substantially uniform amounts of the liquid" means that a relative error of an amount ($Y_l$) of liquid dispensed in a container with respect to a desired amount ($X_l$) of the liquid ($\{(X_l - Y_l)/X_l\} \times 100$) is ±10% or less, ±9% or less, ±8% or less, ±7% or less, ±6% or less, ±5% or less, ±4% or less, ±3% or less, ±2% or less, ±1% or less, or ±0.55% or less.

The "plurality of containers (or products)" in the present disclosure are 50 or more, 60 or more, 70 or more, 80 or more, 90 or more, or 100 or more in number, though the number is not particularly limited to these.

The product that can be produced by the producing method of the present disclosure is, for example, vial products in which cultured cells, liposomes, or medicine-containing particles are dispensed; other examples of the same include cosmetics, and pharmaceutical products.

[Composition]

Further, in another aspect, the present disclosure relates to a composition to be used in the dispensing in the distribution method or the producing method according to the present disclosure, the composition containing a substance for converting a liquid in which particles are dispersed, or a liquid that disperses particles, into a plastic fluid.

The composition according to the present disclosure may be in a solid or powdered state, or in a liquid state.

As the above-described "substance for converting ... into a plastic fluid", the above-described specific compound can be used.

The composition according to the present disclosure may further contain another component, that is, a component other than the above-described substance (specific compound).

Examples of the above-described other component include a cell culturing medium component, and a component of a cell cryopreservation solution, in a case where the particles are cells. The cell culturing medium component can be appropriately selected according to the cells. In the present disclosure, the cell cryopreservation solution may be a medium for cell cryopreservation. Exemplary components of the cryopreservation solution include a cell cryoprotectant agent, an intracellular cryoprotectant agent, and an extracellular cryoprotectant agent.

In another aspect, therefore, the present disclosure relates to a composition containing a cell cryopreservation solution, and a substance for converting the cell cryopreservation solution into a plastic fluid. The composition according to the present disclosure, in one or a plurality of embodiments, is a cell cryopreservation solution in which a "substance for converting the cell cryopreservation solution into a plastic fluid" is dissolved, whereby the cell cryopreservation solution is converted into a plastic fluid. Another composition according to the present disclosure, in one or a plurality of embodiments, is a combination of a cell cryopreservation solution and a "substance for converting the cell cryopreservation solution into a plastic fluid" held in such a form that they are not mixed with each other.

In a case where the above-described other component is a component of a cell cryopreservation solution, a metal ion is preferably added as required, as the specific compound aggregates with the metal ions, or the specific compound forms a three-dimensional network. In one or a plurality of embodiments, the metal ion is a divalent metal ion. In one or a plurality of embodiments, examples of the divalent metal ion include calcium ion, magnesium ion, zinc ion, ferrous ion, and copper ion. In one or a plurality of embodiments, the concentration of the metal ion is 0.1 mM to 300 mM, 0.5 mM to 100 mM, or 0.5 mM to 10 mM.

The present disclosure also relates to one or a plurality of non-limiting embodiments described below:

[1] A method for distributing particles, the method including:

dispersing the particles in a plastic fluid; and
dispensing the plastic fluid containing the particles.

[2] The method according to Item [1], wherein the dispensing is carried out by an automated pipetting system.
[3] The method according to Item [1] or [2], wherein the particles are cells.
[4] A method for producing a product that includes a particle and a container for the particle, the producing method including:
sucking an aliquot of plastic fluid in which the particles are dispersed from a storage part in which the plastic fluid is stored, discharging it into the container, and repeating these processes for a plurality of the containers.
[5] The method according to Item [4], wherein the dispensing is carried out by an automated pipetting system.
[6] The method according to Item [4] or [5], wherein the particles are cells.
[7] A composition to be used in the dispensing in the distribution method according to any one of Items [1] to [3], or the producing method according to any one of Items [4] to [6], the composition containing a substance for converting a liquid in which particles are dispersed, or a liquid that disperses particles, into a plastic fluid.
[8] A composition containing a cell cryopreservation solution, and a substance for converting the cell cryopreservation

solution into a plastic fluid.

Hereinafter, although the following description describes the present disclosure in more detail by way of examples, these are illustrative, and the present disclosure is not limited to these examples.

[EXAMPLE]

An automated pipetting device (FIG. 1) was prepared for dispensing liquid in a liquid reservoir bottle in a storage part 1 into a plurality of vial bottles 4 through pipe lines 2 and a pump 3. Using this automated pipetting device, uniformity of concentration and amount or viability of particles (beads or cells) dispensed into the vial bottles were assessed.

[Assessment Model]

Formula (I) shown below was used as a model for assessing the quality of vial products of cultured cells by dispensing using an automated pipetting device. Formula (I) below is intended to determine an index Y of the number of effective cells in one vial bottle (see FIG. 2).

$$Y = (V/V_t)_{ts,Fs} \cdot (D/D_0)_{ts,Fs} \cdot (P/P_0) \qquad (I)$$

Parameters in Formula (I) are ts and Fs. "ts" represents a period from the start of dispensing to the suction from the liquid reservoir bottle in the storage part (hereinafter, this period is referred to as "suspension time") (h). "Fs" represents a frequency of shaking ($s^{-1}$) of the liquid reservoir bottle.

"$(V/V_t)_{ts,Fs}$" is a relative value of the dispensed amount. "$V_t$" represents a set dispensing amount, and "V" represents a dispensed aliquot amount.

"$(D/D_0)_{ts,Fs}$" is a relative value of the density. "Do" represents the density (the number of particles (or the number of cells) /ml) of an aliquot at the dispensing start time (ts = 0, dispersed state), and "D" represents the density of dispensed aliquot.

"$(P/P_0)$" is a cell viability, which is calculated by the following formula.

$$(P/P_0) = \alpha_{ts,Fs} x \beta_{ts,Fs} x \gamma_{ts,Fs}$$

$$\gamma_{ts,Fs} = n_1/n_0$$

$$\beta_{ts,Fs} = n_2/n_1$$

$$\alpha_{ts,Fs} = n_3/n_2$$

$n_0$: initial viable cell count
$n_1$: viable cell count after dispensing
$n_2$: viable cell count after freezing and rewarming (thawing)
$n_3$: adherent cell count at 24 hours after seeding

$\gamma$ represents a survival ratio determined based on a cell count before stirring and a cell count after stirring.

$\beta$ represents a survival ratio determined based on a cell count before freezing and a cell count after rewarming (thawing).

$\alpha$ represents an adherent cell ratio determined based on an adherent cell count before seeding and an adherent cell count at 24 hours after seeding.

[Test Example 1: Uniformity of Concentration]

Beads and a cryopreservation solution (trade name: STEM-CELLBANKER(R) GMP grade, produced by ZENOAQ) were placed in a liquid reservoir bottle, so that 100 ml of a bead suspension solution having a bead density of $1.0 \times 10^6$ beads/ml was produced. By using an autopipetter and a 50-ml pipet, pipetting inside the bottle was carried

out 10 times at a high speed, so as to make the concentration uniform. The bottle having a uniform concentration was set in a storage part in the automated pipetting device shown in FIG. 1, the tip of a feeding pipe was inserted into the bottle, and the position of the tip of the feeding pipe was adjusted. Thereafter, the suspension solution was dispensed to 100 freezing vials by the automated pipetting device so that each vial contained 1 ml. The bead density in each vial to which the suspension solution was thus dispensed was measured by using an auto cell counter. See FIG. 3.

FIG. 4 shows (D/Do) when sampling was carried out at a position of 5 mm under the surface of the suspension solution at various frequencies of shaking (Fs) and various suspension times (ts).

As illustrated in FIG. 4, at Fs = 0, which means "without stirring", the bead density of the sample at the position of 5 mm under the surface of the suspension solution decreased as time lapsed. This tendency was observed in the cases with the frequencies of shaking of Fs = 0.1 and Fs = 0.4 ($s^{-1}$). It was found that under the conditions of Test Example 1, stirring at a frequency of shaking of Fs = 0.9 $s^{-1}$ or more was required so as to maintain samples dispensed in vials at a uniform density.

(D/Do) was calculated as to each of 100 vials into which suspension solutions stirred at frequencies of shaking of Fs = 0, 0.9, and 1.6 $s^{-1}$ were dispensed. The results are shown in FIG. 5.

As illustrated in FIG. 5, at Fs = 0, which means "without stirring", vials for the latter half of the sampling operation had significantly low bead densities (the encircled part). On the other hand, with stirring at frequencies of shaking of Fs = 0.9 $s^{-1}$ or more, uniform bead densities were maintained in the 100 vials.

[Test Example 2: Uniformity of Dispensed Amount]

With an automated pipetting device that was made capable of pump-sucking while shaking a liquid reservoir bottle, 100 ml of a cryopreservation solution (trade name: STEM-CELLBANKER(R) GMP grade, produced by ZENOAQ) was dispensed to 102 vials over about 30 minutes. The sucking from the liquid reservoir bottle was carried out with a tip of a feeding pipe being fixed at a position of 5 mm above the bottom surface of the bottle.

As to each of 102 vials to which the solution was dispensed, the dispensed amount was measured. The results are shown in FIG. 6.

As shown in FIG. 6 (GG(-)), in cases of the frequencies of shaking of Fs = 0 $s^{-1}$ and Fs = 0.9 $s^{-1}$, the dispensed amount for vials was almost uniform from the start to the end. In a case of the frequency of shaking of Fs = 5.8 $s^{-1}$, however, the dispensed amount for the vials significantly decreased in the latter half of the sampling operation (the encircled part).

Thus, a possibility was indicated that with too great a frequency of shaking (Fs = 5.8 $s^{-1}$), bubbles are sucked into the feeding pipe, which causes the dispensed amount to decrease in the latter half of an operation.

Test Examples 1 and 2 proved that in a case of a common liquid such as a liquid preservation solution, stirring at a speed at a certain level or more is required so as to make the concentration of particles such as beads or cells uniform among vials; however, it is necessary that the stirring is not too fast so that the amount to be dispensed in a vial is made uniform among the vials.

Next, dispensing was carried out in the same manner as that described above except that the cryopreservation solution to which the compound A (deacylated gellan gum, produced by Nissan Chemical Corporation) was added (the concentration of the compound A: 0.06%) was placed in a liquid reservoir bottle, and shaking was not carried out (Fs = 0). Then, the dispensed amount was measured. The results are shown in FIG. 6.

As illustrated in FIG. 6 (GG(+)), in the case where the compound A was added, or in other words, in the case where the cryopreservation solution converted into a plastic fluid was used, the cryopreservation solution could be dispensed in aliquots up to the last vial (the 101st vial). Besides, as illustrated in FIG. 6, in the case where the cryopreservation solution to which the compound A was added, i.e., that was converted into a plastic fluid, was used (GG(+)), the uniformity in the dispensed amounts in the dispensing operation was improved, as compared with the case of the cryopreservation solution to which the compound A was not added (in the case where the cryopreservation solution was not a plastic fluid).

[Test Example 3: Cell Viability]

In place of the beads of Test Examples 1 and 2, hiPS cells cultured in a T-225 flask (D2 strain, StemFit medium) were used (see FIG. 7). Live cells were counted after culturing before dispensing, after dispensing before freezing, and after freezing and rewarming, and adherent cells were counted after 24-hour culturing after freezing and rewarming. Then, a cell viability (P/Po) was calculated.

Influences of the time of shaking applied during a predetermined suspension time (1 h or 4 h) on the viability ($P/P_0$) were confirmed. The results are shown in FIG. 8.

"ts" in the present test example represents a time since the cell suspension solution was prepared with use of a cryopreservation solution until the solution was frozen. In FIG. 8, "0 h" and "0.5 h" as the shaking times in the graph

of ts = 1 h mean that the solution was sampled (dispensed) without being shaken, and that the solution was sampled after being shaken for 30 minutes, respectively, the solutions being frozen at a time of "ts = 1 h". The same applies to the graph of ts = 4 h.

As illustrated in FIG. 8, it is clear that, for example, in a case where the dispensing took four hours, the cell viability (P/Po) of the aliquot dispensed first and that of the aliquot dispensed one to four hours later were significantly different. In other words, it is indicated that even in the same lot, there were vials (products) having significantly different cell viabilities, depending on the period while they were shaken in the dispensing process.

[Production of Plastic Fluid]

Calcium chloride was added to a cryopreservation solution (trade name: STEM-CELLBANKER(R) GMP grade, produced by ZENOAQ) so that the concentration of calcium chloride was 2 mM, and 92.5 ml of the solution was placed in a liquid reservoir bottle. Using a syringe and an injection needle, 7.5 ml of a compound A (deacylated gellan gum, produced by Nissan Chemical Corporation) was added so that the concentration thereof was 0.06%, was mixed by inversion, and was stored overnight in a refrigerator.

[Test Example 4: Uniformity of Concentration (Plastic Fluid)]

Beads and a compound-A-added cryopreservation solution were placed in a liquid reservoir bottle, so that 100 ml of a bead suspension solution having a bead density of $1.0 \times 10^6$ beads/ml, was produced. By using an autopipetter and a 50-ml pipet, pipetting inside the bottle was carried out 10 times at a high speed, so as to make the concentration of the beads in the bottle uniform. The bottle having a uniform concentration of the beads was set in a storage part in the above-described automated pipetting device, the tip of a feeding pipe was inserted into the bottle, and the position of the tip of the feeding pipe was adjusted to a position of 5 mm under the surface of the suspension solution. Thereafter, the suspension solution was dispensed to 100 freezing vials by the automated pipetting device so that each vial contained 1 ml. The bead density in each vial to which the suspension solution was thus dispensed was measured by using an auto cell counter. See FIG. 9.

Bead densities (D/Do) were calculated as to the following: vials to which the suspension solution obtained by adding the compound A to the cryopreservation solution was dispensed; and vials to which the suspension obtained without addition of the compound A was dispensed, without shaking (Fs = 0). The results are shown in FIG. 10.

As illustrated in FIG. 10, in a case where Fs = 0, which means "without shaking", and the compound A was not added, vials for the latter half of the sampling operation had significantly low bead densities ($D/D_0$). On the other hand, even though shaking was not carried out, in a case where the compound A was added, the decrease in the bead density ($D/D_0$) was suppressed.

[Test Example 5: Cell Viability (Plastic Fluid)]

Dispensing, freezing, rewarming (thawing), and culturing were carried out with predetermined suspension times ts (0 to 4 h) in the same manner as that in Test Example 3, except that the liquid reservoir bottle was not shaken (Fs = 0), and a cryopreservation solution was converted to a plastic fluid by using $CaCl_2$ and the compound A ($CaCl_2$: 2mM, Compound A: 0.06%). A viability $\gamma$ (%) determined based on a cell count before stirring and a cell count after stirring, a viability $\beta$ (%) determined based on a cell count before freezing and a cell count after rewarming (thawing), and an adherent cell ratio $\alpha$ (%) determined based on an adherent cell count before seeding for recultivation and an adherent cell count at 24 hours after the same were calculated. Further, a double acceleration $\mu$ (h) in recultivation was measured. These results are compared with those in a case where the compound A was not used (the solution was not converted to a plastic fluid), and are shown in FIG. 11.

Besides, based on observation of recultivation, comparison was carried out between a case where the cryopreservation solution was converted to a plastic fluid with use of the compound A, and a case where the compound A was not added (in the case where the cryopreservation solution was not converted to a plastic fluid). The results are shown in FIG. 12.

As illustrated in FIGs. 11 and 12, even in a case where a cryopreservation solution converted to a plastic fluid was used, the above-described parameters ($\alpha$, $\beta$, $\gamma$, and $\mu$) were identical to those in a case where a cryopreservation solution not converted to a plastic fluid was used. No cytotoxicity was observed in a case where a plastic fluid was used.

From the above, it is recognized that the use of a plastic fluid in dispensing a cell-dispersed cryopreservation solution can suppress the unevenness of the dispensed amount for an aliquot and the unevenness of the cell density without stirring/shaking, and further, enables a dispensing operation that can suppress the unevennesses while maintaining the cell quality (viability, etc.).

[Test Example 6: Cell Viability (Plastic Fluid)]

Plastic fluids (SCB-GG(+) and SS-GG(+)) were produced by the same procedure in the above-described plastic fluid production, using cryopreservation solutions shown in Table 1 below, $CaCl_2$, and the compound A (deacylated gellan gum, produced by Nissan Chemical Corporation).

| (Table 1) | Cryopreservation solution | $CaCl_2$ (mM) | Compound A (%) |
|---|---|---|---|
| SCB-GG(-) | STEM-CELLBAN KER® (produced by ZENOAQ) | - | - |
| SCB-GG(+) | | 0.5 | 0.06 |
| SS-GG(-) | Stem Sure® Calcium Free (produced by Wako) | - | - |
| SS-GG(+) | | 0.5 | 0.06 |

In the fluids SCB-GG(+) and SS-GG(+), hiPS cells (1383D2 strain) were dispersed, and dispensing, freezing, rewarming (thawing), and culturing were carried out with predetermined suspension times ts (0 to 4 h), without shaking of the liquid reservoir bottle (Fs = 0). A viability $\gamma$ (%) determined based on a cell count before dispensing and a cell count after dispensing, a viability $\beta$ (%) determined based on a cell count before freezing and a cell count after rewarming (thawing), an adhesion rate $\alpha$ (%) determined based on an adherence cell count before seeding for recultivation and an adherent cell count at 24 hours after the same, and a cell potential P/Po(= $\alpha\times\beta\times\gamma$) were calculated. The results are shown in FIG. 13.

Performed was the same operation as that described above except that the compound A was not used (the solution was not converted to a plastic fluid, i.e., SCB-GG(-) and SS-(GG(-)) were used). The results are shown in FIG. 13. It should be noted that in the case where the compound A was not used (in the case of SCB-GG(-) and SS-(GG(-))), the dispensing was carried out while the liquid reservoir bottle was being shaken (Fs = 0.90 $s^{-1}$) so that the cell concentration was made uniform.

As illustrated in FIG. 13, in any case of the cryopreservation solutions, the following was recognized: it is more likely that converting the same to a plastic fluid tends to result in suppressing a decrease in the cell potential that can be caused by dispensing, rewarming and the like.

## Claims

1. A method for distributing particles, the method comprising:

   dispersing the particles in a plastic fluid; and
   dispensing the plastic fluid containing the particles.

2. The method according to claim 1,
   wherein the dispensing is carried out by an automated pipetting system.

3. The method according to claim 1 or 2,
   wherein the particles are cells.

4. A method for producing a product that includes a particle and a container for the particle, the producing method comprising:
   dispensing a plastic fluid in which the particles are dispersed, from a storage part in which the plastic fluid is stored, to a plurality of the containers.

5. The method according to claim 4,
   wherein the dispensing is carried out by an automated pipetting system.

6. The method according to claim 4 or 5,
   wherein the particles are cells.

7. A composition to be used in the dispensing in the distribution method according to any one of claims 1 to 3, or the producing method according to any one of claims 4 to 6,

the composition containing a substance for converting a liquid in which particles are dispersed, or a liquid that disperses particles, into a plastic fluid.

**8.** A composition containing:

a cell cryopreservation solution; and
a substance for converting the cell cryopreservation solution into a plastic fluid.

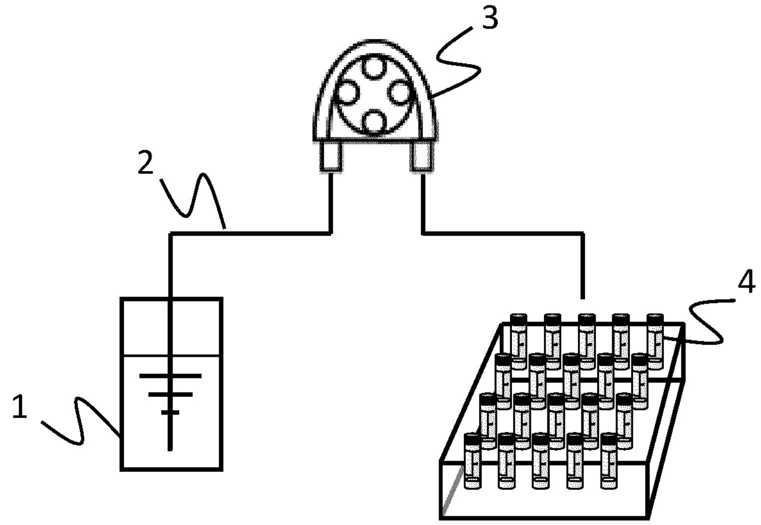


FIG.1

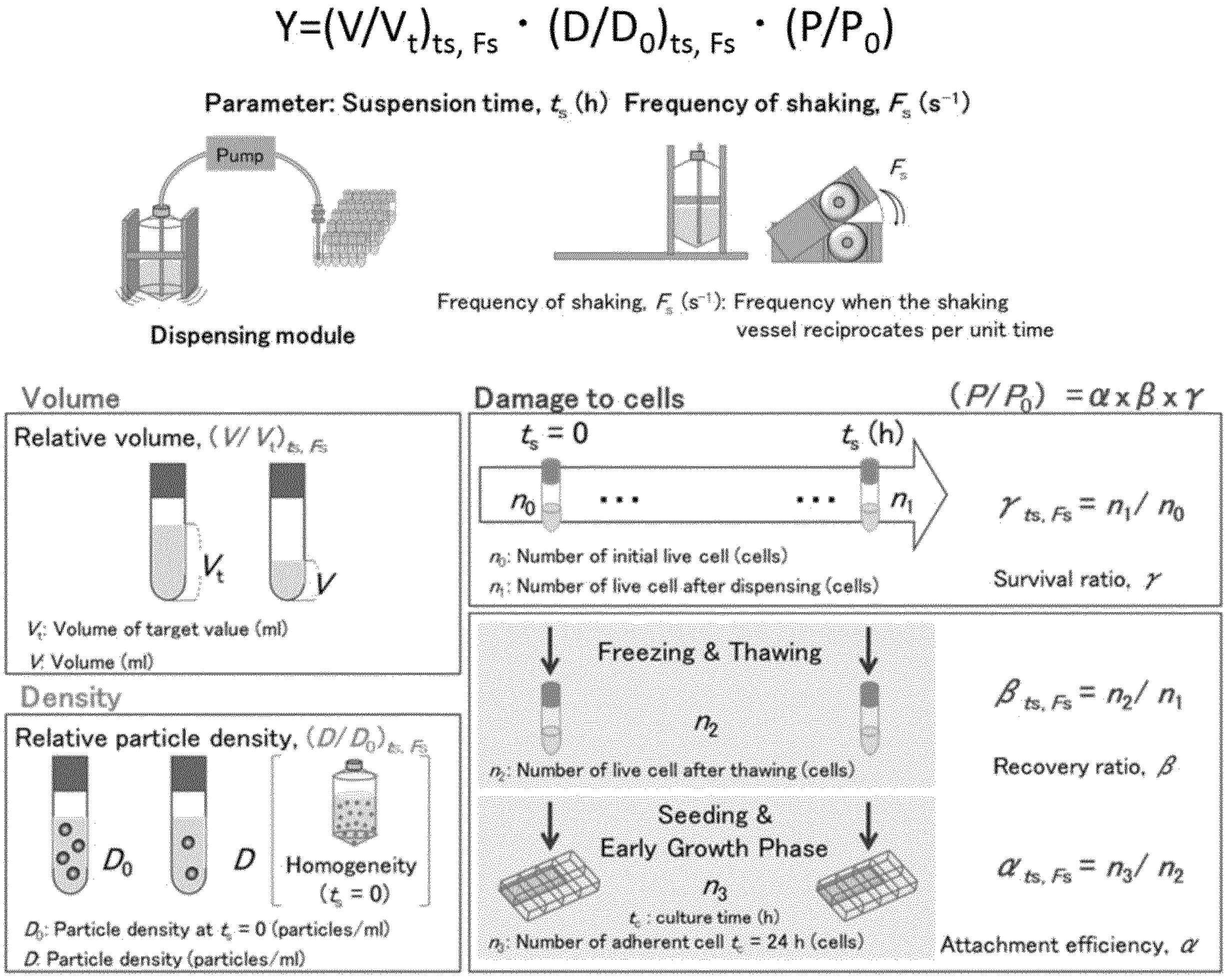
Y=(V/Vt)ts, Fs · (D/D0)ts, Fs · (P/P0)
Parameter: Suspension time, ts (h) Frequency of shaking, Fs (s-1)
Pump
Dispensing module
Fs
Frequency of shaking, Fs (s-1): Frequency when the shaking vessel reciprocates per unit time
Volume
Relative volume, (V/Vt)ts, Fs
Vt
V
Vt: Volume of target value (ml)
V: Volume (ml)
Density
Relative particle density, (D/D0)ts, Fs
D0
D
Homogeneity (ts = 0)
D0: Particle density at ts = 0 (particles/ml)
D: Particle density (particles/ml)
Damage to cells
(P/P0) = α x β x γ
ts = 0
ts (h)
n0
n1
γ ts, Fs = n1/ n0
n0: Number of initial live cell (cells)
n1: Number of live cell after dispensing (cells)
Survival ratio, γ
Freezing & Thawing
n2
β ts, Fs = n2/ n1
n2: Number of live cell after thawing (cells)
Recovery ratio, β
Seeding & Early Growth Phase
n3
α ts, Fs = n3/ n2
tc : culture time (h)
n3: Number of adherent cell tc = 24 h (cells)
Attachment efficiency, α

FIG. 2

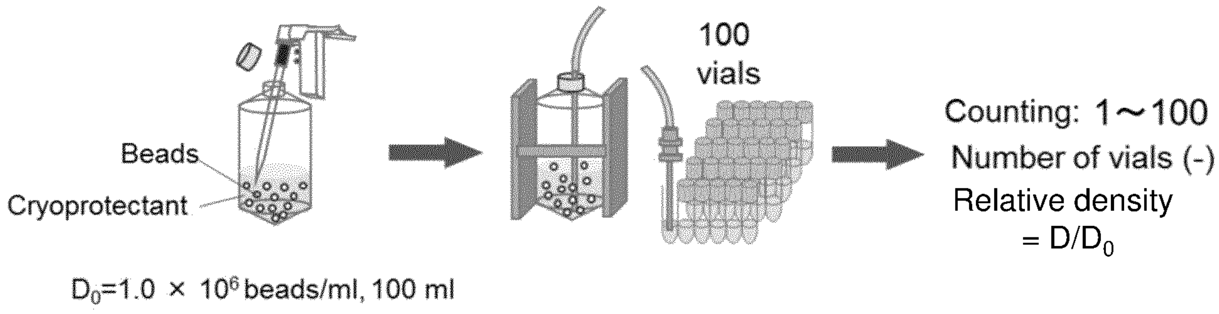
Beads
Cryoprotectant
D0=1.0 × 10^6 beads/ml, 100 ml
100 vials
Counting: 1～100
Number of vials (-)
Relative density
= D/D0

FIG. 3

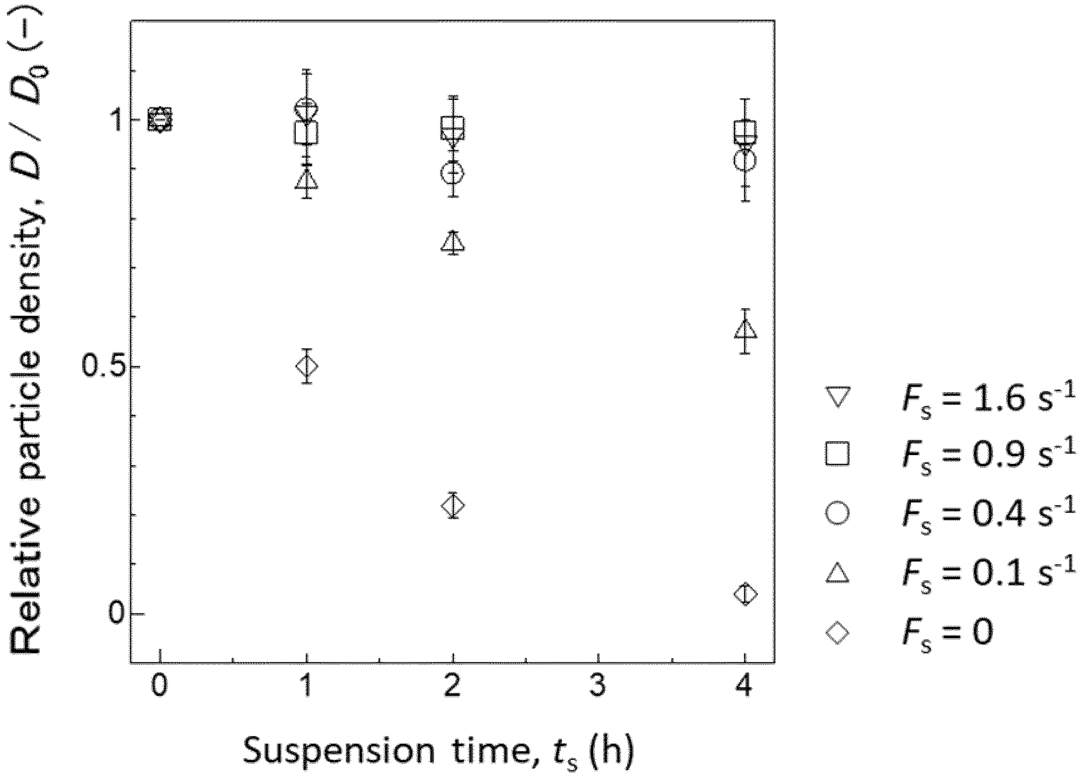
Relative particle density, D / D0 (-)
1
0.5
0
0
1
2
3
4
Suspension time, ts (h)
Fs = 1.6 s-1
Fs = 0.9 s-1
Fs = 0.4 s-1
Fs = 0.1 s-1
Fs = 0

FIG. 4

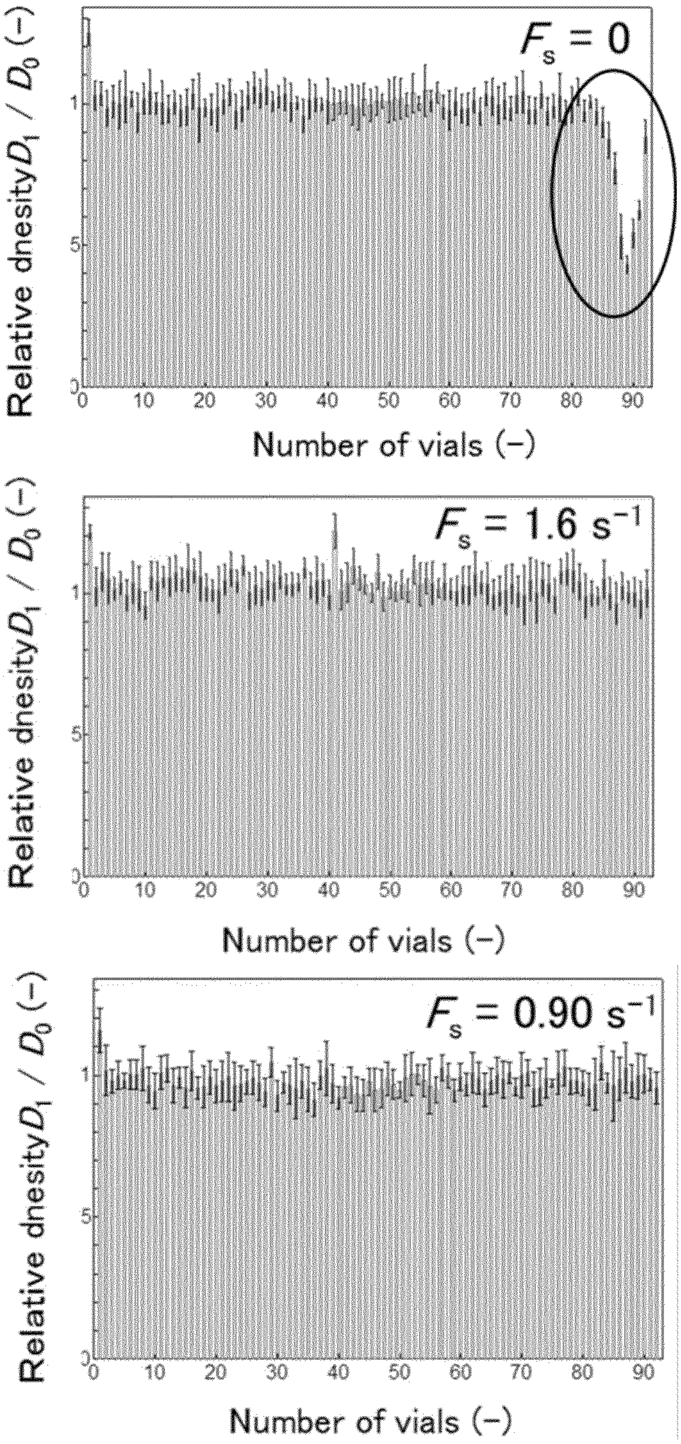


·Analysis

Relative density was calculated as shown below:

Relative density = $D_1/D_0$

$D_1$: Bead concentration in vial after dispensing (beads/ml)

$D_0$: Initial bead concentration in bottle (beads/ml)

FIG. 5

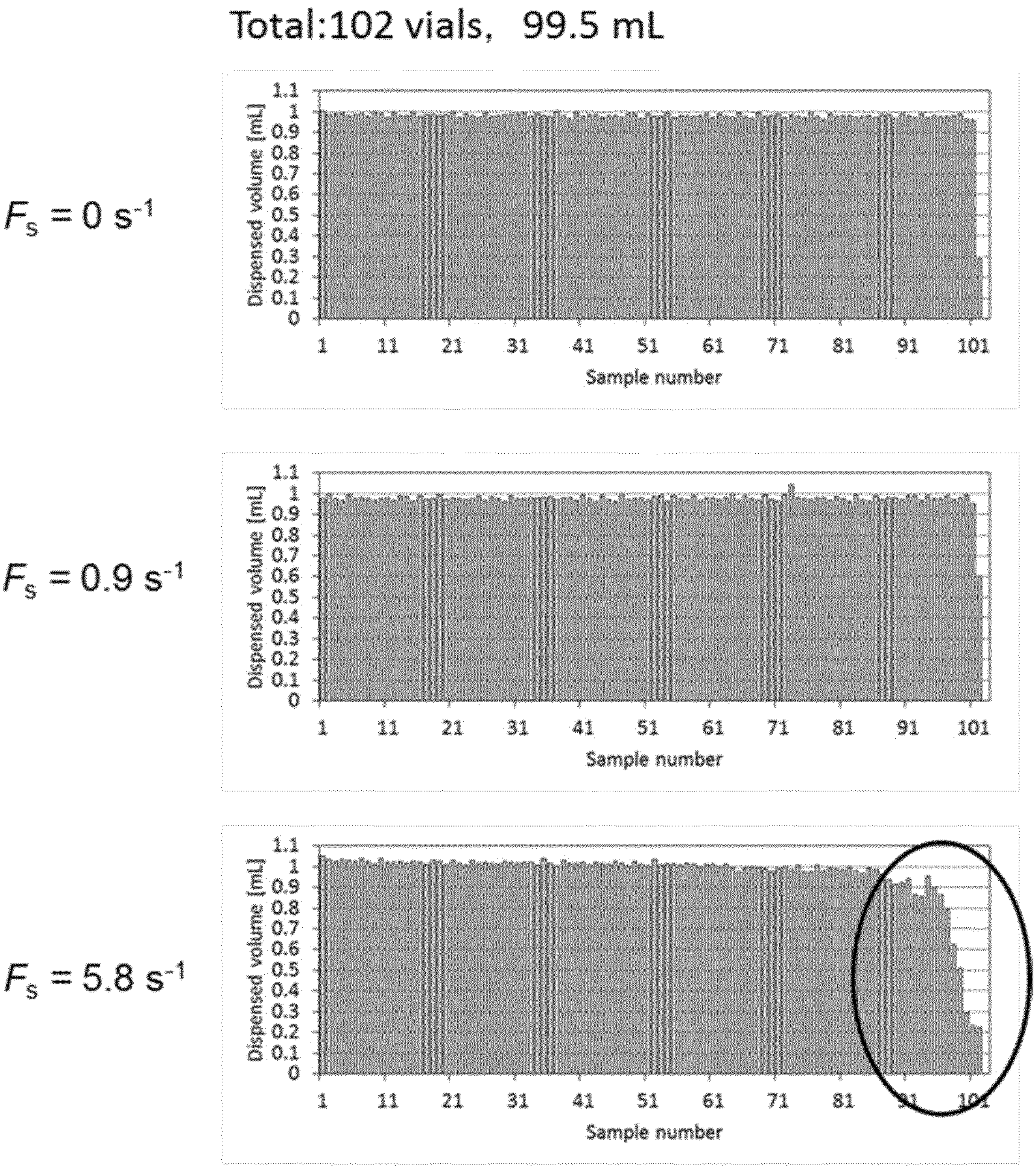
Total:102 vials, 99.5 mL
$F_s = 0\ s^{-1}$
$F_s = 0.9\ s^{-1}$
$F_s = 5.8\ s^{-1}$
Dispensed volume [mL]
Sample number
1.1
1
0.9
0.8
0.7
0.6
0.5
0.4
0.3
0.2
0.1
0
1
11
21
31
41
51
61
71
81
91
101

FIG. 6

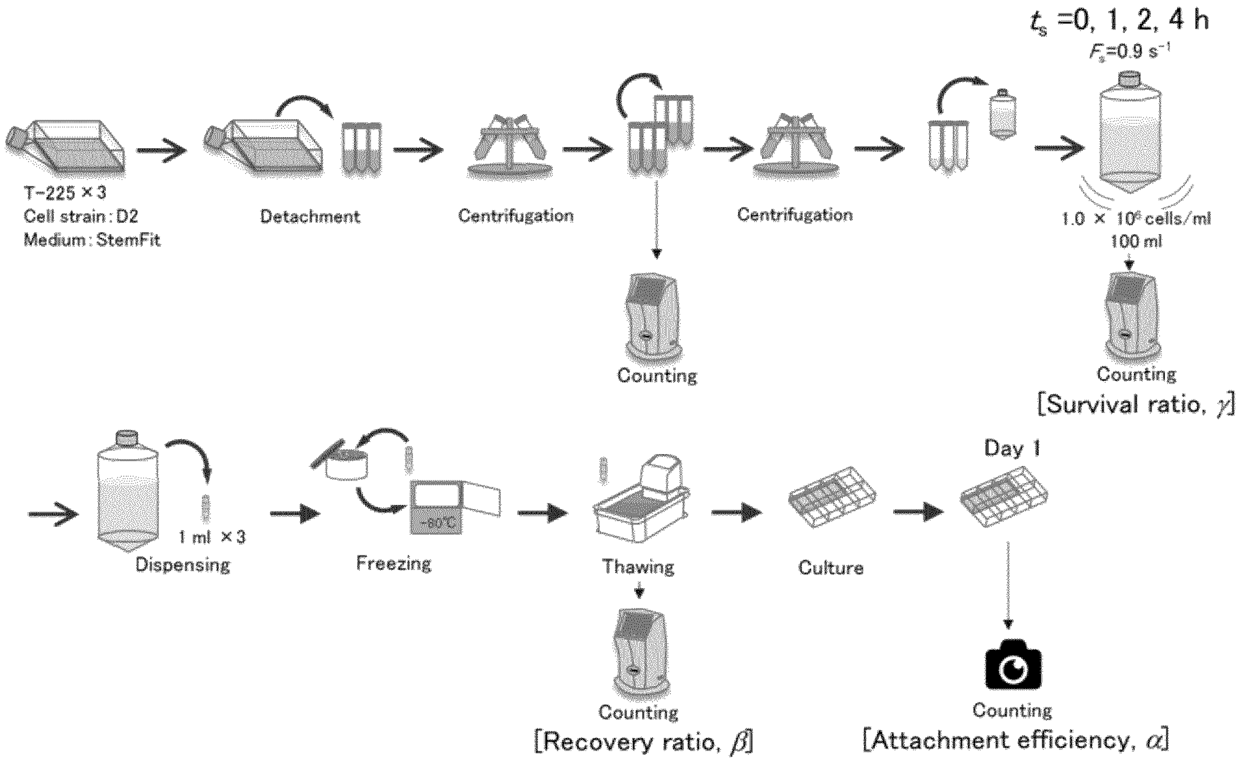

t_s =0, 1, 2, 4 h
F_s=0.9 s^-1
T-225 × 3
Cell strain: D2
Medium: StemFit
Detachment
Centrifugation
Counting
Centrifugation
1.0 × 10^6 cells/ml
100 ml
Counting
[Survival ratio, γ]
1 ml × 3
Dispensing
-80℃
Freezing
Thawing
Counting
[Recovery ratio, β]
Culture
Day 1
Counting
[Attachment efficiency, α]


FIG. 7

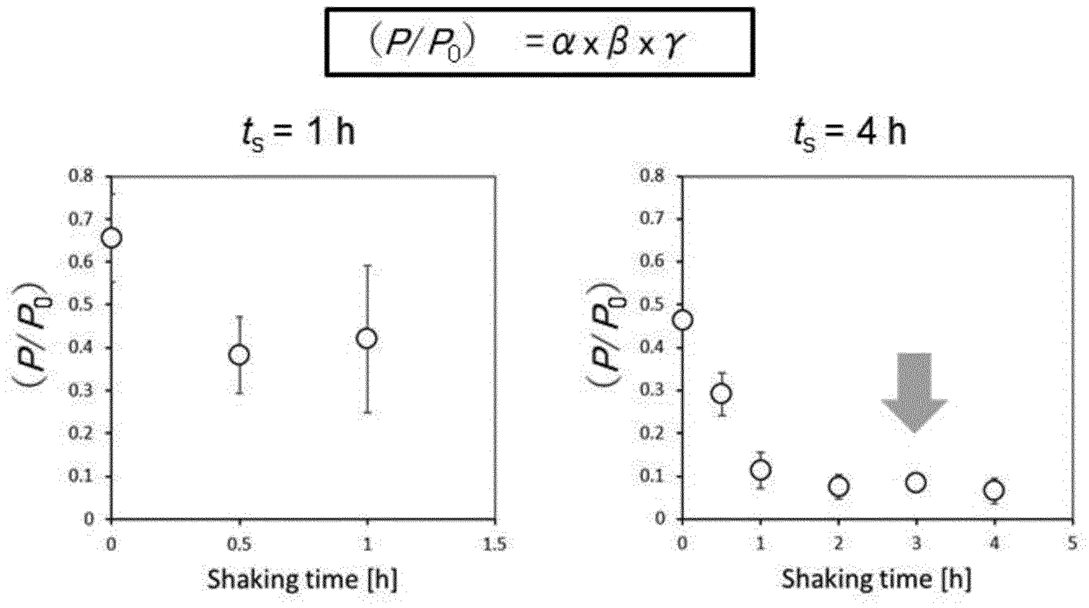
(P/P0) = α x β x γ
ts = 1 h
ts = 4 h
(P/P0)
0.8
0.7
0.6
0.5
0.4
0.3
0.2
0.1
0
0.5
1
1.5
2
3
4
5
Shaking time [h]

FIG. 8

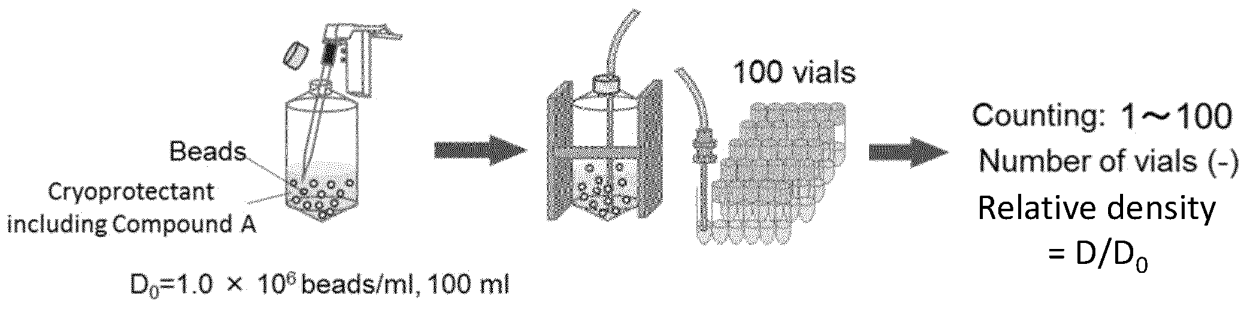
Beads
Cryoprotectant including Compound A
D0=1.0 × 10^6 beads/ml, 100 ml
100 vials
Counting: 1~100
Number of vials (-)
Relative density
= D/D0

FIG. 9

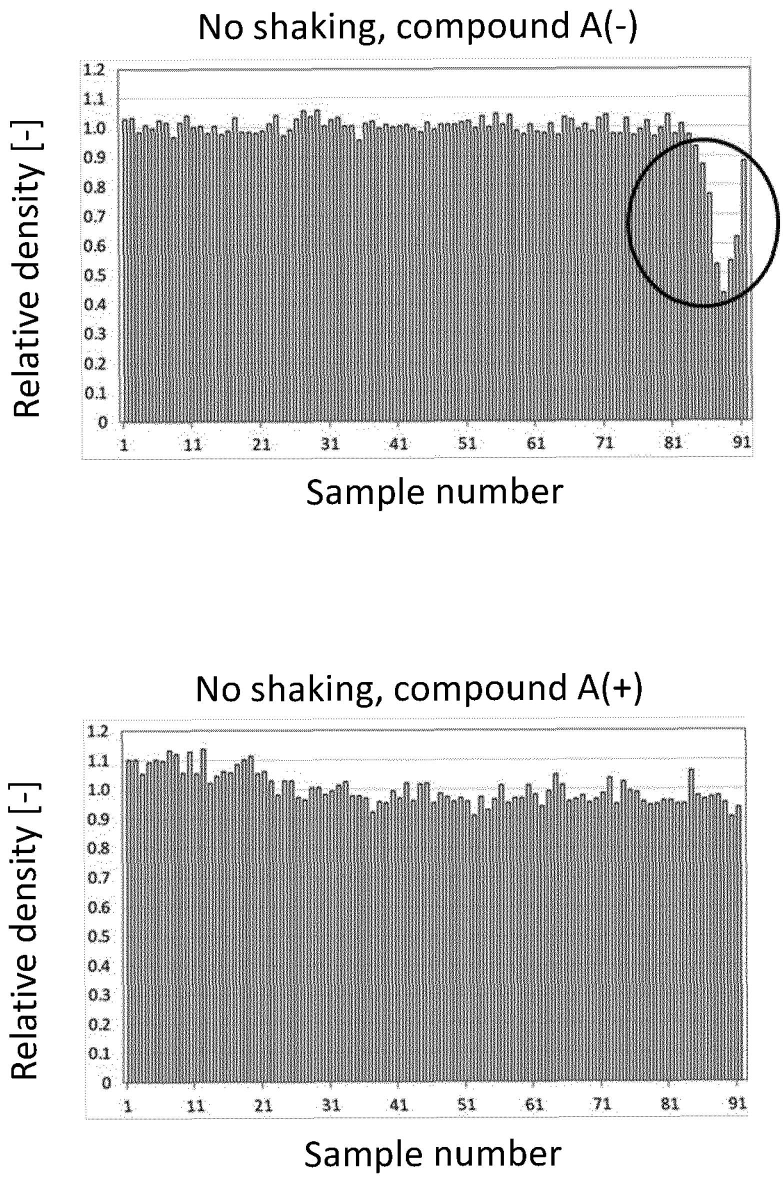
No shaking, compound A(-)
Relative density [-]
1.2
1.1
1.0
0.9
0.8
0.7
0.6
0.5
0.4
0.3
0.2
0.1
0
1
11
21
31
41
51
61
71
81
91
Sample number
No shaking, compound A(+)
Relative density [-]
1.2
1.1
1.0
0.9
0.8
0.7
0.6
0.5
0.4
0.3
0.2
0.1
0
1
11
21
31
41
51
61
71
81
91
Sample number

FIG. 10

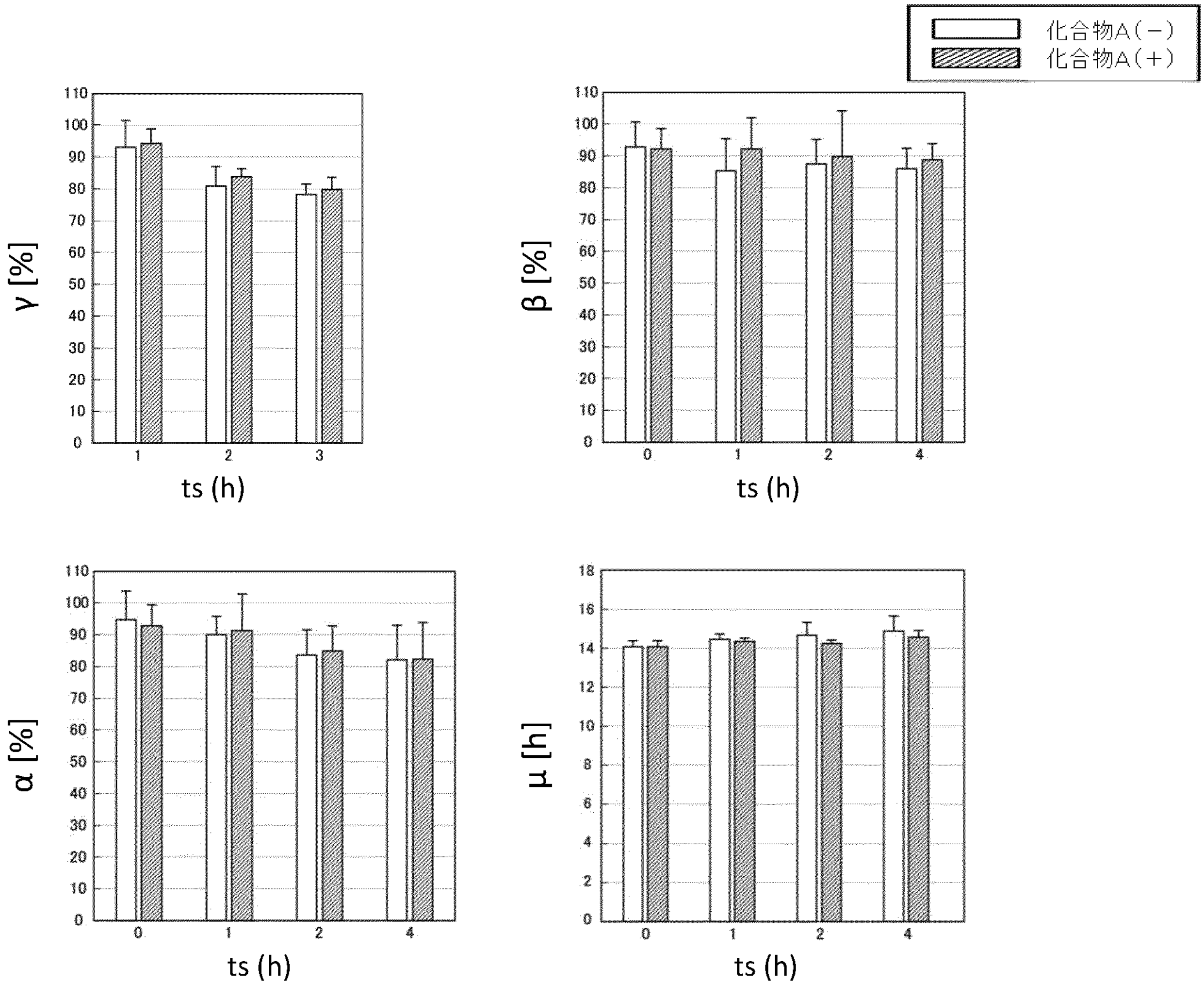


FIG. 11

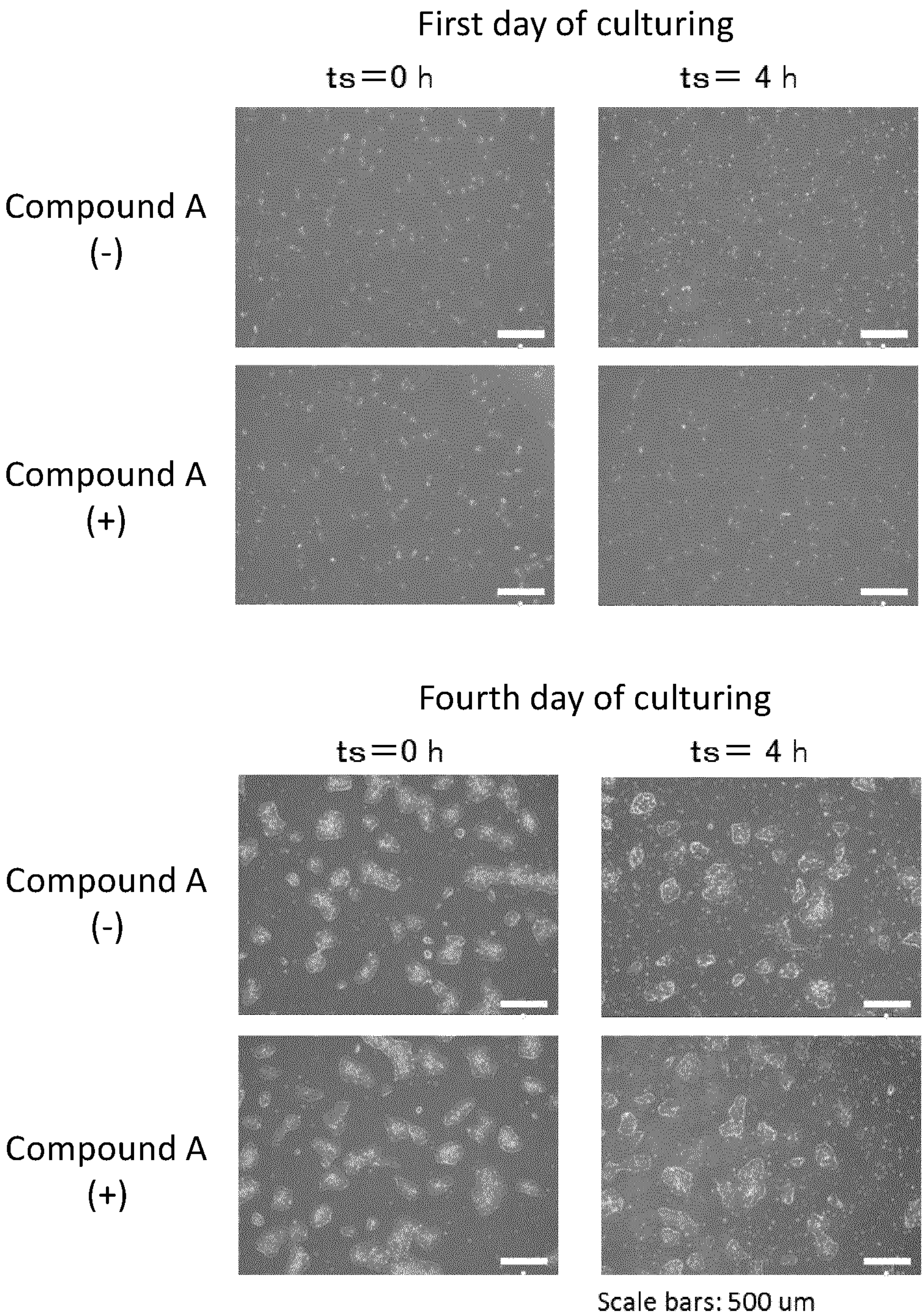
First day of culturing
ts=0 h
ts= 4 h
Compound A (-)
Compound A (+)
Fourth day of culturing
ts=0 h
ts= 4 h
Compound A (-)
Compound A (+)
Scale bars: 500 um

FIG. 12

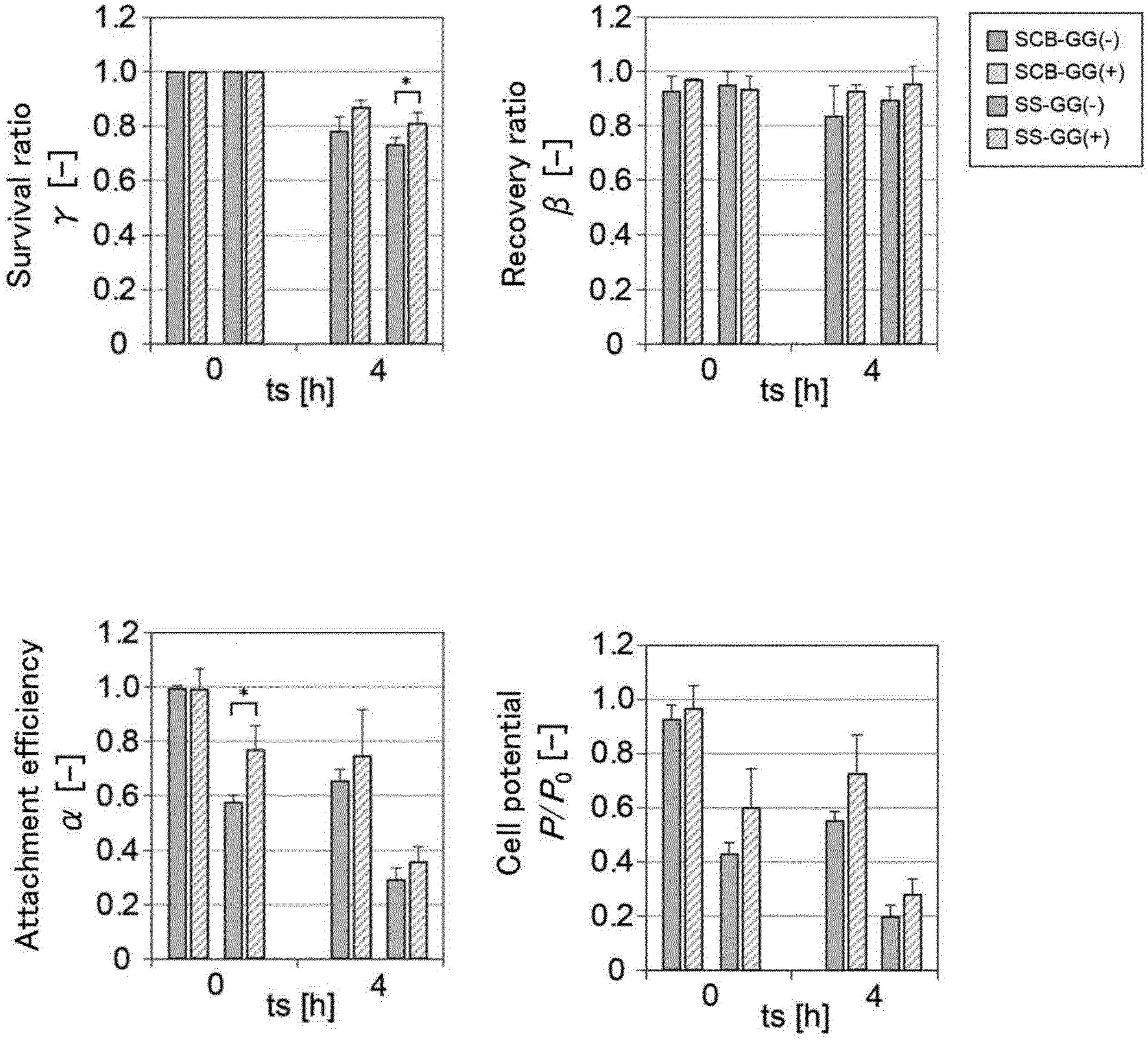


Test for significant difference (*p < 0.05) using Student's *t*-test.

FIG. 13

## INTERNATIONAL SEARCH REPORT

International application No. PCT/JP2019/031860

**A. CLASSIFICATION OF SUBJECT MATTER**

Int.Cl. G01N1/00(2006.01)i, B01J4/02(2006.01)i, C12N5/04(2006.01)i, C12N5/07(2010.01)i, G01N1/28(2006.01)i, G01N35/10(2006.01)i, C12M1/00(2006.01)n, C12M1/24(2006.01)n, C12M1/26(2006.01)n, C12N1/04(2006.01)n

According to International Patent Classification (IPC) or to both national classification and IPC

**B. FIELDS SEARCHED**

Minimum documentation searched (classification system followed by classification symbols)

Int.Cl. G01N1/00-1/44, G01N35/10, B01J4/02, C12N5/04, C12N5/07, C12M1/00, C12M1/24, C12M1/26, C12N1/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2014/017513 A1 (SAN CHEMICAL INDUSTRIES, LTD., KYOTO UNIVERSITY) 30 January 2014, paragraphs [0033], [0034], [0046], [0082]-[0085] & EP 2878664 A1 (paragraphs [0046]-[0059], [0074], [0142]-[0144]) & CA 2879624 A1 & AU 2013294057 A1 & KR 10-2015-0038190 A & CN 104640974 A & HK 1210499 A1 & RU 2015106003 A & BR 112015001620 A2 & IL 236878 A & TW 201420755 A | 1-7<br>8 |
| Y | JP 2003-202334 A (ASAHI MEDICAL CO., LTD.) 18 July 2003, paragraph [0027] (Family: none) | 8 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 29 October 2019 (29.10.2019) | 12 November 2019 (12.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/031860

C (Continuation). DOCUMENTS CONSIDERED TO BE RELEVANT

| Category* | Citation of document, with indication, where appropriate, of the relevant passages | Relevant to claim No. |
|---|---|---|
| A | US 2014/0106348 A1 (KYOTO UNIVERSITY) 17 April 2014, entire text, all drawings & US 2017/0175077 A1 | 1-8 |
| A | US 2014/0360269 A1 (ROCHE DIAGNOSTICS OPERATIONS, IN. C) 11 December 2014, paragraphs [0041], [0049]-[0053], [0081], [0089], [0100], [0115], fig. 7, 17 & EP 2810717 A1 & CN 104237492 A & HK 1201918 A1 | 1-8 |
| A | JP 2005-312398 A (JAPAN SCIENCE AND TECHNOLOGY AGENCY) 10 November 2005, paragraphs [0032]-[0045] (Family: none) | 1-8 |
| A | WO 2016/121896 A1 (NISSAN CHEMICAL INDUSTRIES, LTD., CHIBA UNIVERSITY) 04 August 2016, entire text, all drawings & US 2018/0008646 A1 & EP 3252151 A1 & CN 107208059 A & KR 10-2017-0125027 A & TW 201636423 A | 1-8 |

Form PCT/ISA/210 (continuation of second sheet) (January 2015)

**INTERNATIONAL SEARCH REPORT**

International application No.
PCT/JP2019/031860

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention in claims 1 and 3 is disclosed in paragraphs [0033], [0034], [0046], [0082]-[0085] of WO 2014/017513 A1. Also, an invention-specifying feature described in claim 2 is merely a simple well-known feature. Thus, the invention in claims 1-3 does not have a special technical feature. Therefore, the inventions in independent claims 1, 4, and 8 do not have an identical or corresponding special technical feature, and do not comply with the requirement of unity of invention.

Next, when examining the number of inventions, a difference between the main invention in claims 1-3 and the invention in claims 4-7 is merely a design modification involved in the specific application of the technology, and thus the invention in claims 4-7 is added to the main invention. Meanwhile, the invention in claim 8 does not have an invention-specifying feature specified to distribution of particles, and thus cannot be added to the main invention. Thus, the invention in claim 8 is classified as invention 2. Therefore, the main invention (claims 1-7) and invention 2 (claim 8) are described in the claims.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009058288 A
- JP 2012509485 A
- JP 2008102052 A
- JP 5668021 B
- WO 2014017513 A